# EUROPÄISCHE PATENTSCHRIFT

(11) **EP 1 234 572 B1**
(45) Veröffentlichungstag und Bekanntmachung des Hinweises auf die Patenterteilung: **30.10.2013**
(21) Anmeldenummer: 02405096.5
(22) Anmeldetag: 11.02.2002
(51) Int. Cl.: A61Q 19/00, A61Q 19/02, A61K 8/97

(54) **Isoflavon-Aglykone enhaltende Hautbehandlungsmittel**
Skin treatment compositions containing isoflavone aglycones
Produits de soin pour la peau contenant de l'isoflavone aglycone

(30) Priorität: 26.02.2001 CH 3492001
(43) Veröffentlichungstag der Anmeldung: 28.08.2002
(73) Patentinhaber: Mibelle AG Cosmetics, 5033 Buchs (CH)
(72) Erfinder: Zülli, Fred, Dr., 5024 Küttigen (CH); Schmid, Daniel, 5200 Brugg (CH); Muggli, Reto, 4600 Olten (CH); Hanay, Christiane, 5000 Aarau (CH)
(74) Vertreter: Rottmann, Maximilian

(56) Entgegenhaltungen:
- EP-A- 0 692 250
- WO-A-97/46208
- WO-A-98/56373
- WO-A-99/36050
- WO-A-99/47118
- DE-A- 4 432 947
- PATENT ABSTRACTS OF JAPAN vol. 2000, no. 01, 31. Januar 2000 (2000-01-31) & JP 11 269066 A (KAO CORP), 5. Oktober 1999 (1999-10-05)
- PATENT ABSTRACTS OF JAPAN vol. 008, no. 071 (C-217), 3. April 1984 (1984-04-03) & JP 58 225004 A (ICHIMARU FUARUKOSU KK), 27. Dezember 1983 (1983-12-27)
- PATENT ABSTRACTS OF JAPAN vol. 2000, no. 13, 5. Februar 2001 (2001-02-05) & JP 2000 302667 A (KOBE TENNENBUTSU KAGAKU KK), 31. Oktober 2000 (2000-10-31)
- PATENT ABSTRACTS OF JAPAN vol. 1998, no. 13, 30. November 1998 (1998-11-30) & JP 10 226642 A (KIKKOMAN CORP), 25. August 1998 (1998-08-25)

## Beschreibung

Die vorliegende Erfindung betrifft Hautbehandlungsmittel gemäss dem Oberbegriff von Anspruch 1.

Die menschliche Haut besteht bekanntlich aus zwei Schichten, der äusseren, dünneren Epidermis und der darunterliegenden, dickeren Dermis. Die Epidermis enthält als hauptsächlichen Zelltyp Keratinozyten, die in der äussersten Zone verhornt sind und so die Haut vor Austrocknung und mechanischen wie chemischen Einflüssen schützen. Die Dermis ist reich an Strukturkomponenten, wie den Kollagen- und Elastin-Proteinen sowie den Proteoglykanen, Zucker/Protein-Komplexen. Diese Strukturkomponenten werden von Fibroblasten-Zellen gebildet und geben der Haut die nötige Dicke und Festigkeit sowie Elastizität.

Das Phänomen Hautalterung zeigt sich durch Bildung von Hautrunzeln und allgemein Verdünnung der Haut und Abnahme deren Elastizität und Festigkeit. Hautalterung ist einerseits ein chronologischer Vorgang, kann andererseits aber durch äussere Faktoren wie z. B. UV-Strahlung beschleunigt werden. UV-Strahlung führt zu reaktiven Oxidationsprodukten, die Mechanismen in Gang setzen, welche auch bei der chronologischen Hautalterung ablaufen. Diese in der Dermis spielenden Mechanismen sind ein verminderter Aufbau von Strukturelementen sowie ein beschleunigter Abbau der bestehenden. Bei Frauen erfolgt nach der Menopause eine ruckartige Hautalterung. Das Phänomen Menopause entsteht wegen eines drastischen Rückgangs der Produktion des weiblichen Geschlechtshormons Östrogen. Es wird in diesem Zusammenhang daher von "hormonbedingter Hautalterung" gesprochen.

Als Wirkstoffe gegen die UV-induzierte Hautalterung werden Antioxidantien eingesetzt. Gebräuchliche Wirkstoffe gegen die Hautalterung allgemein sind α -Hydroxy-Säuren sowie Retinsäure. Diese Substanzen erzeugen jedoch unangenehme Nebeneffekte, wie Hautspannung und Hautirritationen. Ein anderer Ansatz in der Therapie der Hautalterung ist, Substanzen einzusetzen, die in die Regulierung der Synthese von Dermis-Strukturelementen eingreifen. Erwünschenswert sind Wirkstoffe, die den Aufbau dieser Strukturproteine fördern und/oder deren Abbau verhindern. Dafür bekannt sind Östrogene oder Östrogen-ähnliche Substanzen.

Mehrere Studien haben gezeigt, dass durch eine Therapie mit oralem Östrogen (Hormon-Ersatz-Therapie) Dicke und Elastizität der Haut bei Frauen nach der Menopause erhalten werden konnten. Auch Therapien mit äusserlich angewandtem Östrogen waren erfolgreich in der Bekämpfung der abrupten Hautalterung nach der Menopause. Die ständige Aufnahme von Östrogen birgt jedoch ein erhöhtes Risiko für die Entwicklung von Brust- und Gebärmutterkrebs. Seit kurzem wurden synthetische Östrogen-ähnliche Substanzen eingeführt (zum Beispiel Raloxifen), die ohne Krebsrisiko sein sollen.

Isoflavone werden zur Zeit intensiv untersucht als natürliche Alternative zur Östrogen-Ersatz-Therapie. Isoflavone stellen eine Klasse der Pflanzenöstrogene dar. Sie sind in Struktur und Funktion dem menschlichen Östrogen sehr ähnlich. Im Gegensatz zu diesem weisen sie jedoch kein Krebsrisiko auf, sondern wirken sogar antikanzerogen und werden als solche auch in Functional Food Produkten vermarktet.

Soyabohnen sind sehr reichhaltig an Isoflavonen. Sie enthalten die Isoflavon-Aglykone Genistein, Daidzein und Glycitein sowie die entsprechenden β-Glycoside Genistin, Daidzin und Glycitin. Nicht-fermentierte Soyaprodukte enthalten hauptsächlich die polaren und somit wasserlöslichen Glycoside. Wird Soya fermentiert, werden die Zucker durch bakterielle Enzyme abgespalten und die Glycoside mehrheitlich in die biologisch aktiven Aglykone überführt. Nur die Aglykone haben eine Östrogen-ähnliche Aktivität. Untersuchungen mit Zellkulturen zeigten, dass Isoflavone den Aufbau von Kollagen stimuliert [Kawashima et al., Biochemical Pharmacology, Bd. 51 (1996), Seite 133] sowie die Produktion von Kollagen-abbauenden Enzymen (Matrix-Metalloproteinasen) reduziert [Shao et al., Anticancer Research, Bd. 18 (1998), Seite 1435]. Genistein ersetzt somit die Funktionen im Hautaufbau des nach der Menopause fehlenden Östrogens. Isoflavone üben auch beim Mann einen positiven Effekt auf die Haut aus. Indem sie den Aufbau von Dermisstrukturen fördern, wirken sie der auch bei Männern vorkommenden altersbedingten Hautverdünnung entgegen. Das weibliche Geschlechtshormon Östrogen kann beim Mann nicht eingesetzt werden. Isoflavone wirken je nach Gewebe östrogenartig (Knochen, Blutkreislaufsystem, Gehirn) oder aber antiöstrogenartig (Brust, Uterus) [Maroulis, Annals of the New York Academy of Sciences, Bd. 900 (2000), Seite 413)]. Sie zeigen demnach bei Männern keine unerwünschte Hormoneffekte.

Es sind bereits Patente und Patentanmeldungen über den Einsatz von Isoflavonen in kosmetischen Mitteln veröffentlicht. In JP-58225004 und JP-7157494 werden Isoflavone als Mittel zur Hautaufhellung (Whitening-Mittel) beschrieben. In DE-4432947 werden Isoflavone zur Behandlung der Haut gegen Couperose, Besenreiser, Melanome, Alopecie, Akne, Fetthaut und Pigmentflecken, als Haarwuchsmittel und Radikalfänger beschrieben. In US-5824702 wird Genistein als vorbeugendes Mittel zum Schutz gegen UV-induzierte Hautschäden beschrieben. In WO-99/04747 ist das Isoflavon Resveratrol als Mittel zur Therapie von gealterter und Licht-geschädigter Haut beschrieben. WO-99/36050 zeigt die Wirkung von Isoflavonen gegen UV-induzierte Unterdrückung des Immunsystems sowie gegen UV-induzierte Hautschädigung allgemein. In FR-2782919 wird behauptet, dass eine Mischung von Retinsäure mit Isoflavonen das Entstehen von Zeichen der Hautalterung verzögern könne. Darin wird dargelegt, dass die Mischung einen synergistischen Effekt aufweise, wobei der Effekt darin bestehe, dass der Abbau von Dermis-Strukturproteinen reduziert werde. US-6060070 beschreibt den Einsatz von Isoflavonen gegen Hautalterung bei Männern wie Frauen, beruhend auf der Östrogen-ähnlichen Aktivität der Isoflavone. **Aus der** DE 44 32 947 A1 **ist ebenfalls ein Mittel zur Behandlung der Haut bekannt, das als aktiven Wirkstoff ein Isoflavon enthält.**

Die oben genannten Veröffentlichungen unterscheiden nicht die Anwendung von Isoflavonen in Form von Zucker-Derivaten oder von Aglykonen. Aus folgenden Gründen ist dies, vor allem bei topischer Anwendung, ein entscheidender Punkt. Als Zucker-Derivate haben die Isoflavone keinen physiologischen Effekt. Isoflavone liegen in der Pflanze natürlicherweise als Zucker-Derivate vor. Zur Aktivierung müssen die Zucker abgespalten werden. Werden Isoflavone oral, z.B. als Nahrungsergänzungsmittel, aufgenommen, müssen sie vorher nicht aktiviert werden, da die Zucker im Darm durch hydrolytische Enzyme der Darmzellen und der Darmflora abgespalten werden. Werden die Isoflavone jedoch in Kosmetikprodukten eingesetzt, müssen sie vorher aktiviert werden, d.h. in die Aglykon-Form gebracht werden, weil auf der Haut keine aktiviert werden, d.h. in die Aglykon-Form gebracht werden, weil auf der Haut keine hydrolytischen Enzyme vorhanden sind. Als Zucker-Derivate würden die Isoflavone auch nicht in die tiefere Hautschicht, die Dermis, eindringen, weil die von der Epidermis gebildete Fettschicht, nur die apolaren, wasserunlöslichen Aglykone passieren lässt. Diese vollkommen wasserunlöslichen Aglykone können jedoch nur durch Kombination mit lösungsvermittelnden Stoffen in kosmetische Produkte eingebracht werden.

Die vorliegende Erfindung hat sich nunmehr die Aufgabe gestellt, wirksame Hautbe-handlungsmittel zur Behandlung der Zeichen von Hautalterung allgemein, der abrupten Hautalterung der Frauen in und nach der Menopause, zur Behandlung von Zellulitis und Akne, zur Vergrösserung und Festigung der weiblichen Brust und zur Hautaufhellung zur Verfügung zu stellen.

Diese Aufgabe wird gelöst durch die im Kennzeichen des Anspruchs 1 genannten Massnahmen. Bevorzugte Ausführungsformen sind in den abhängigen Ansprüchen 2 bis 5 umschrieben.

Die Erfindung beruht somit auf einer neuartigen Formulierung **mit einem Gehalt an mindestens einem** Isoflavon-Aglykonen **in einer biologische aktiven Form als Wirkstoff und einem wässrigen Algenextrakt, sowie** einem Trägersystem. Letzteres besteht aus dem Phospholipid Lecithin, das sich in wässriger Lösung zu Liposomen formiert. Die wasserunlöslichen Aglykone siedeln sich in der Lecithin-Doppellipid-Membran der Liposomen an. Derart in Liposomen eingebaut können Aglykone stabil in wässrige kosmetische Endprodukte einformuliert werden. Werden die Produkte auf die Haut aufgetragen, verdunstet der Wasseranteil allmählich, die Liposomen fallen auseinander, das Lecithin verschmilzt mit der hauteigenen Fettschicht und die freigewordenen Aglykone können durch diese Fettschicht in tiefere Hautschichten eindringen.

Bei den erfindungsgemäss einsetzbaren Isoflavonen handelt es sich um eine oder mehrere Substanzen aus der Gruppe Genistein, Daidzein, Glycitein, Formononetin, Tectorigenin, Irigenin, Biochanin A, O-Desmethylangolensin, Equol, Orobol, Santal, Pratensin und Apiosylpuerarin.

Im Weiteren wird die Herstellung eines Aglykon-Wirkstoffes aus den Soya-Isoflavonen Genistin, Daidzin und Glycitin beschrieben.

Die Entstehung der Zellulitis ist hauptsächlich durch die geschlechtsspezifische anatomische Struktur der Haut und den Einfluss von Geschlechtshormonen bestimmt. Beim Mann überkreuzen sich in den oberen subkutisschichten Bindegewebssepten scherenartig, welche die Fettzellen klammerartig einschliessen. Beim Zusammenpressen der Haut werden die Fettkammern durch das Bindegewebsgitter zurückgehalten. Die Epidermis-Korium-Schicht ist dicker, die Subkutisschicht dünner als bei der Frau. Bei dieser besteht die obere Subkutisschicht aus vertikalen Fettzellkammern, die durch radiär verlaufende Bindegewebssepten röhrenförmig voneinander getrennt sind. Die obere Begrenzung dieser Fettröhren bildet die dünnere und schwächere Epidermis-Korium-Schicht, die sich beim Zusammenpressen der Haut polsterartig hochwölbt. Etwa ab dem 30. Lebensjahr wird die Epidermis-Korium-Schicht dünner, die elastischen und kollagenen Fasern werden schwächer bzw. weniger, die Subkutis dagegen dicker, was ein polsterartiges Hautrelief begünstigt.

Algenextrakte gewinnen eine immer grössere Bedeutung als alternative Rohstoffquellen für den Einsatz in der Kosmetik. Besonders die blaugrüne Mikroalge aus der Gattung *Spirulina* ist bekannt für ihren hohen Anteil an mehrfach ungesättigten Fettsäuren, essentiellen Aminosäuren, Mineralstoffen und natürlichen Antioxidantien, wie α -Tocopherol und β-Carotin. Algenextrakte werden als Radikalfänger und Feuchtigkeitsfaktor in Anti-Aging-Kosmetikprodukten eingesetzt. Die Wirksamkeit von Algenextrakten in der Behandlung von Zellulitis beruht auf einer fettlösenden Aktivität und einem verbesserten Abtransport von Schlacken.

Der Einsatz von Isoflavonen in der Zellulitis-Therapie ist ein völlig neuer Ansatz. Es ist bekannt, dass eine Hormon-Ersatz-Therapie mit Östrogen zu einer verbesserten Hautstruktur führt. Eine topische Behandlung von Zellulitis mit Östrogen ist jedoch fraglich, da die Zellulitis ja ursprünglich durch Östrogen induziert wird. Es ist weiter bekannt dass die Östrogen-ähnlichen Isoflavone in Brust und Uterus anti-östrogene Aktivität aufweisen. Diese Tatsache, zusammen mit der Tatsache, dass Genistein den Aufbau von Kollagen stimuliert und die Produktion von Kollagen-abbauenden Enzymen (Matrix-Metalloproteinasen) reduziert, sprechen für ein hohes Potential von Isoflavonen in der Zellulitis-Behandlung. Eine topische Behandlung mit Isoflavonen sollte zur Verdickung und Verstärkung der Dermisschicht führen, wodurch polsterartiges Hochwölben der Fettzellkammern reduziert werden kann.

Genistein bewirkt im menschlichen Körper eine Reduktion des Fettgewebes. Diese Oesterogen-unabhängige Wirkung ist ein zusätzlicher, wichtiger Mechanismus in der Behandlung von Zellulitis durch Genistein. Der Abbau des Fettes erfolgt weil Genistein das Enzym Phosphodiesterase hemmt [Kuppusamy und Das, Biochemistry and Pharmacology, Bd. 44, Seite 1307]. Die Hemmung führt dazu, dass mehr zyklisches Adenosinmonophosphat in der Zelle vorliegt, einem Botenstoff innerhalb der Zelle, welcher das Enzym Lipase zum Abbau von Fett anregt. Ein allgemeiner Abbau von Fettgewebe erfolgt auch, weil Genistein die Vermehrung von Vorläufer-Fettzellen hemmt [Harmon und Harp, American Journal of Physiological Cell Physiology, Bd. 280, Seite C807].

Es existiert eine umfangreiche Literatur über die Brustkrebs-hemmenden Eigenschaften der Isoflavone. Doch Isoflavone können auch den Stoffwechsel von gesundem Gewebe beeinflussen. Zur Zeit werden mehrere Produkte mit Pflanzenextrakten (z.B. aus Black Cohosh oder *Pueraria mirifica*), die Pflanzenöstrogene enthalten, zur Vergrösserung und Festigung der Brust angeboten. Die Wirkung der Produkte soll die Folge einer Stimulierung der Bildung von Brustgewebe und Vergrösserung des Milchganges sein. Eine wissenschaftliche Publikation [McMichael-Phillips et al., American Journal of Clinical Nutrition, Bd. 68 Suppl. (1998), Seite 1431] zeigte, dass erhöhte Genistein- und Daidzein-Blutwerte nach Soya-Zusatz-Ernährung signifikant das Wachstum von Brustgewebe stimulierten.

Der Befund dass Isoflavone das Wachstum von gesundem Brustgewebe stimulieren zusammen mit der Tatsache, dass sie die Dermisschicht der Brusthaut verdicken und verstärken, sprechen für den Einsatz von Isoflavonen zur Vergrösserung und Verstärkung der weiblichen Brust.

*Acne vulgaris,* die häufigste Hauterkrankung überhaupt, entsteht durch die Hormonumstellung während der Pubertät, manchmal auch während der Periode oder der Schwangerschaft. Entscheidend in der Pathogenese ist eine übermässige Talgproduktion. Diese Talgdrüsen werden durch Androgene, die sogenannten "männlichen" Hormone, aktiviert. So ist ein erhöhter Androgen-Spiegel häufig die Ursache von Akne. Akne kann durch östrogene Substanzen, welche den Androgen-Spiegel normalisieren, wirksam bekämpft werden. Hormone sind in kosmetischen Produkten jedoch nicht erlaubt, und Östrogene weisen zudem Nebeneffekte, wie erhöhtes Krebsrisiko, auf. Isoflavone sind natürliche, östrogenähnliche Substanzen, die ebenfalls den Androgen-Spiegel normalisieren sollten.

Isoflavone weisen neben der Fett abbauenden Wirkung weitere nicht-hormonähnliche Wirkungen auf. Diese sind bedingt durch die Eigenschaft der Isoflavone, gewisse Enzyme, sogenannte Protein-Kinasen, zu hemmen. Das Enzym Protein-Kinase spielt eine entscheidende Rolle bei der Synthese von Farbpigmenten in den Melanoma-Zellen. Die Protein-Kinase aktiviert das Enzym Tyrosinase, das eine Schlüsselposition in der Synthese der Farbpigmente hat. Die bisher gebräuchlichen Substanzen zur Aufhellung der Haut wirken in den meisten Fällen durch Hemmung des Enzyms Tyrosinase. Isoflavone stellen im Bereich Hautaufhellung eine neue Wirkstoffgruppe dar.

Es sind bereits Soya-lsoflavon-Wirkstoffe auf dem Markt. Diese enthalten die Pflanzenöstrogene jedoch in der biologisch nicht aktiven Gykosid-Form. Dies ist nicht von Bedeutung, wenn die Wirkstoffe in Functional-Food-Produkten als Ernährungs-Zusatzstoffe eingesetzt werden, da die Enzyme der Darmflora die Glykoside in die aktiven Aglykone umsetzen. Werden diese Isoflavon-Wirkstoffe jedoch auf der Haut angewendet bleiben sie wirkungslos. Demgegenüber betrifft die vorliegende Erfindung einen Isoflavon-Wirkstoff, der erstmals Pflanzenöstrogene in ihrer biologisch wirksamen Aglykon-Form enthält. Zusätzlich können die wasserunlöslichen Aglykone in eine liposomale Struktur eingebaut werden, eine galenische Form also, die es erlaubt, den Wirkstoff in kosmetische Formulierungen einzubringen. Die liposomale Struktur gewährt den Aglykonen zudem ein grosses Eindringungsvermögen in die Hautzellen.

Der liposomale Aglykon-Wirkstoff lässt sich dadurch herstellen, dass Soya-lsoflavon-Material mit einem β-Glucosidase-Enzym während 24 bis 500 Stunden bei 20 bis 60°C behandelt wird. Die dabei gebildeten wasserunlöslichen Aglykone werden danach durch Zentrifugation oder Filtration abgetrennt. Die Aglykone können dann in 100%igem Alkohol aufgelöst werden. Für den Einbau in Liposomen wird die Alkohollösung mit einer wässrigen Lecithin-Dispersion homogenisiert.

Der liposomale Aglykon-Wirkstoff lässt sich auch dadurch herstellen, dass als Ausgangsmaterial eine fermentierte Soyafraktion verwendet wird. Durch die Fermentation werden die Isoflavon-Glycoside in die entsprechenden Aglykone überführt. Ein geeignetes Ausgangsmaterial ist zum Beispiel der Presskuchen, der nach der Moromi-Fermentation bei der Soyasaucen-Herstellung anfällt. Die Aglykone können mit 100%igem Isopropanol extrahiert werden. Für den Einbau in Liposomen wird die Isopropanollösung mit einer wässrigen Lecithin-Dispersion homogenisiert oder gerührt.

Weiter lässt sich der liposomale Aglykon-Wirkstoff auch dadurch herstellen, dass als Ausgangsmaterial Soya-Melasse eingesetzt wird. Soya-Melasse ist ein Abfallprodukt, das bei der Produktion von Soya-Protein-Konzentrat anfällt. Bei der Herstellung von Protein-Konzentrat wird Soya-Mehl zuerst mit einer wässerigen Alkohollösung extrahiert, um Bitterstoffe und unerwünschte Geruchstoffe mit der typisch bohnigen Note zu entfernen. Bei der Rezyklierung des Alkohols fällt die Soya-Melasse an, ein Konzentrat von Zuckern, Bitterstoffen und Isoflavonen. Für die Herstellung des liposomalen Aglykon-Wirkstoffes kann verfahren werden wie für das Soya-lsoflavon-Material beschrieben.

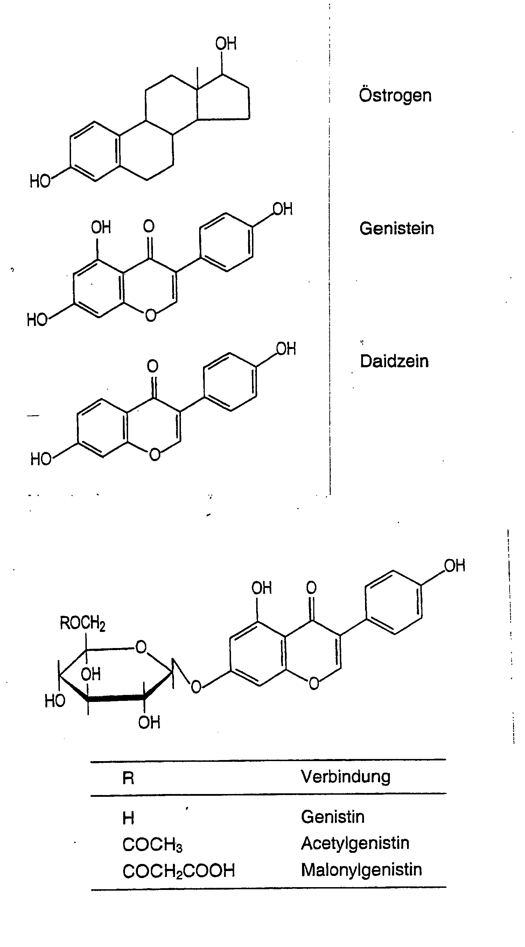

Die Formeln einiger der im Zusammenhang mit der vorliegenden Erfindung interessierenden Verbindungen sind in der beiliegenden Zeichnung dargestellt.

Die folgenden Beispiele und Rezepturen sollen den Erfindungsgegenstand näher erläutern.

Alle im Folgenden genannten Zahlen sind - wo nichts anderes angegeben ist - Gewichts-Prozent. Die Bezeichnung der Inhaltsstoffe entspricht hauptsächlich der INCI-Nomenklatur (International Cosmetics Ingredients), wie sie zur Kennzeichnung kosmetischer Wirk- und Hilfsstoffe bestimmt ist.

### Beispiele

### Beispiel 1

### Herstellung eines Soya-Isoflavon-Aglykon-Wirkstoffes in Alkohol

### (a) Aus einer an Isoflavonen angereicherten Soyafraktion

Als Ausgangsmaterial wurde eine an Isoflavonen angereicherte Soyafraktion eingesetzt. Für die Hydrolyse wurden 50 g dieses Materials in 1 I Kaliumsorbat (0,6%ig, pH 5,0) aufgenommen und mit 300 mg β-Glucosidase bei 37°C während 4 Tagen behandelt. Die ausgefallenen, wasserunlöslichen Aglykone wurden durch Filtration abgetrennt und anschliessend zweimal mit Wasser gewaschen. Zur Extraktion der Aglykone wurde das Filtrat in 420 ml Alkohol aufgelöst und 2 Stunden bei 25°C gerührt. Verbleibendes alkoholunlösliches Material wurde durch Filtration abgetrennt. Die Analyse des Alkohol-Extraktes durch HPLC (C18-Säule) zeigte, dass 86 % des ursprünglichen Genistin-Glykosides und 50 % des ursprünglichen Daidzin-Glykosides als deren Aglykone wiedergewonnen werden konnten.

### (b) Aus Presskuchen, der bei der Soyasaucen-Herstellung nach Koji- und Moromi-Fermentation anfällt

Analyse des Presskuchen durch HPLC (C18-Säule) zeigte, dass dieser 0,07 % Genistein und 0.04 % Daidzein enthielt. Vor der Isolation der Isoflavon-Aglykone wurde der Presskuchen mit Wasser gewaschen. Dazu wurden 400 g Presskuchen in 4 I Wasser aufgenommen und 2 Stunden bei 25°C gerührt. Das wasserunlösliche Material wurde durch Filtration abgetrennt, in 2 I Alkohol aufgenommen und 2 Stunden bei 25°C gerührt. Der Alkoholextrakt wurde durch Filtration vom Feststoff abgetrennt. Dieser Extraktionsprozess wurde einmal wiederholt. Die Alkoholextrakte wurden vereinigt und in einem Rotavapor®-Vakuumverdampfer auf einen Hundertstel eingeengt.

### (c) Aus Soya Melasse, einem Nebenprodukt der Herstellung von Soya Protein Konzentrate

Für die Hydrolyse wurden 100 g Soya-Melasse in 1 I Kaliumsorbat (0,6%ig, pH 5,0) aufgenommen und mit 300 mg β-Glucosidase bei 37°C während 4 Tagen behandelt. Dann wurde wie oben unter (a) beschrieben verfahren.

### Beispiel 2

### Herstellung eines Soya-lsoflavon-AglykonlLiposomen-Wirkstoffes

Zur Herstellung der Aglykon-Liposomen-Lösung, wurden 10 ml Soya-lsoflavon-Aglykon-Wirkstoff in Alkohol, hergestellt nach einem der Verfahren wie in Beispiel 1 unter (a) bis (c) beschrieben, mit 10 ml 50%iger Lecithin-Lösung in Alkohol gemischt, in 80 ml Wasser eingerührt und 5-mal bei 1200 Bar (120 Pa) homogenisiert. Der Partikeldurchmesser der Liposomen betrug 120±20 nm.

### Beispiel 3

### Herstellung einer Soya Isoflavon-Aglykon/Algenextrakt-Wirkstoff-Kombination

| | |
|---|---|
| Wässriger Algenextrakt aus *Spirulina platensis* | 40,0 % |
| Soya-Isoflavon-Aglykon/Liposomen-Wirkstoff in Alkohol (2 % Aglykone) | 20,0 % |
| Polysorbate 80 | 20,0 % |
| Konservierungsmittel, Wasser | ad 100,0 % |

### Rezepturen

### 1. Antizellulitis-Gel

| | |
|---|---|
| Glucose | 4,0 % |
| Aluminium Starch Octenyl Succinate | 1,5 % |
| Soya-Isoflavon-Aglykon/Algenextrakt-Wirkstoff-Kombination (0,25 % Aglykone) | 5,0 % |
| Polysorbate 20 | 0,6 % |
| Carbomer | 0,5 % |
| *Ginkgo biloba*-Extrakt | 0,5 % |
| Konservierungsmittel, Natronlauge, Parfum, Aqua | ad 100,0 % |

### 2. Antizellulitis-Creme

| | |
|---|---|
| Caprylic/Capric Triglyceride | 12,0 % |
| Hydrogenated Coco-Glyceride | 3,0 % |
| Polyglyceryl-3-Methylglucose Distearate | 3,0 % |
| Soya-Isoflavon-Aglykon/Algenextrakt-Wirkstoff-Kombination (0,4 % Aglykone) | 5,0 % |
| Glyceryl Stearate - | 6,0 % |
| Cetylalkohol | 1,0 % |
| Glyceryl Polymethacrylate | 1,0 % |
| Stearyl Alcohol | 1,0 % |
| *Ginkgo biloba*-Extrakt | 0,5 % |
| Konservierungsmittel, Parfum, Aqua | ad 100,0 % |

### 3. Antizellulitis-Intensiv-Konzentrat

| | |
|---|---|
| Soya-Isoflavon-Aglykon/Algenextrakt-Wirkstoff-Kombination | 3,0 % |
| Pentylene Glycol | 2,0 % |
| Carnitine | 0,2 % |
| Caffeine | 0,1 % |
| *Ruscus aculeatus*-Extrakt | 0,1 % |
| Butylene Glycol | 2,0 % |
| Glycerin | 2,0 % |
| Polysorbate-20 | 1,0 % |
| Xanthan Gum | 0,3 % |
| Konservierungsmittel, Parfum, Aqua | ad 100,0 % |

### 4. Antizellulitis-2-Phasenbad

| | |
|---|---|
| Paraffinum Liquidum | 20,0 % |
| Sodium Laureth Sulfate | 8,4 % |
| Propylene Glycol | 8,0 % |
| Cocamidopropyl Betaine | 3,0 % |
| Sodium Chloride | 2,5 % |
| Glycerin | 2,0 % |
| Isohexadecane | 1,0 % |
| Soya-lsoflavon-Aglykon/Algenextrakt-Wirkstoff-Kombination | 3,0 % |
| Carnitine | 0,2 % |
| Caffeine | 0,1 % |
| *Ruscus aculeatus*-Extrakt | 0,1 % |
| Konservierungsmittel, Parfum, Aqua | ad 100,0 % |

### 5. Antizellulitis-Hydro-Intensiv-Massagecreme

| | |
|---|---|
| Isononyl Isononanoate | 4,0 % |
| Glycerin | 4,0 % |
| Paraffinum Liquidum | 4,0 % |
| Arachidyl Glucoside, Arachidyl Alcohol | 5,0 % |
| Soya-Isoflavon-Aglykon/Algenextrakt-Wirkstoff-Kombination | 3,0 % |
| Carnitine | 0,2 % |
| Caffeine | 0,1 % |
| *Ruscus aculeatus*-Extrakt | 0,1 % |
| Squalane | 2,0 % |
| Myristyl Glucoside, Myristyl Alcohol | 2,0 % |
| Cyclomethicone | 2,0 % |
| Butylene Glycol | 2,0 % |
| Carbomer | 0,3 % |
| Konservierungsmittel, Parfum, Aqua | ad 100,0 % |

### 6. Gesichtscreme gegen Zeichen der Hautalterung für Frauen nach der Menopause

| | |
|---|---|
| Octyldodecanol | 5,0 % |
| Paraffinum Liquidum | 3,0 % |
| Isopropyl Isostearate | 3,0 % |
| Cetyl Alcohol | 2,5 % |
| Stearyl Alcohol | 2,0 % |
| Dicaprylyl Ether | 2,0 % |
| Palmitic/Stearic Acid | 2,0 % |
| Polyglyceryl-3-Methylglucose Distearate | 2,0 % |
| Propylene Glycol | 2,0 % |
| Soya-Isoflavon-Aglykon/Liposomen-Wirkstoff (0,2 % Aglykone) | 5,0 % |
| Glycerin | 1,0% |
| Xanthan Gum | 0,2 % |
| Konservierungsmittelsmittel, Parfum, Wasser | ad 100,0 % |

### 7. Körpercreme gegen Zeichen der Hautalterung für Frauen nach der Menopause

| | |
|---|---|
| Cetearyl Glucoside | 5,0 % |
| Diispropyl Dimer Dilinoleate | 5,0 % |
| Paraffinum liquidum | 5,0 % |
| Glycerin | 2,0 % |
| Butyrospermum Parkii | 2,0 % |
| Soya-Isoflavon-Aglykon/Liposomen-Wirkstoff (0,2 % Aglykone) | 0,1 % |
| Dimethicone | 1,0 % |
| Squalane | 0,5 % |
| Carbomer | 0,2 % |
| Konservierungsmittel, Parfum, Aqua | ad 100,0 % |

### 8. Serum gegen Zeichen der Hautalterung für Frauen nach der Menopause

| | |
|---|---|
| Dicaprylyl Ether | 5,0 % |
| Glycerin | 3,0 % |
| Distarch Phosphate | 2,5 % |
| Trilaureth-4 phosphate | 2,5 % |
| Dimethicone | 3,0 % |
| Butyl Alcohol | 1,0% |
| Soya-Isoflavon-Aglykon/Liposomen-Wirkstoff (0,2 % Aglykone) | 20,0 % |
| Carbomer | 0,5 % |
| Konservierungsmittel, Natronlauge, Parfum, Aqua | ad 100,0 % |

### 9. Brustcreme zur Vergrösserung und Festigung der Brüste

| | |
|---|---|
| Dicaprylyl Ether | 4,0 % |
| Isononyl Isononanoate | 3,0 % |
| Arachidyl Glucoside | 3,0 % |
| Octyldodecanol | 3,0 % |
| Myristyl Glucoside | 2,0 % |
| Soya-lsoflavon-Aglykon/Liposomen-Wirkstoff (0,2 % Aglykone) | 5,0 % |
| Dimethicone | 1,0 % |
| Carbomer | 0,5 % |
| Konservierungsmittel, Natronlauge, Parfum, Aqua | ad 100,0 % |

### 10. Anti-Akne-Gesichtsemulsion

| | |
|---|---|
| Polyacrylamide | 3,5 % |
| Hydrogenated Polyisobutene | 3,5 % |
| Glycerin | 2,0 % |
| Propylene Glycol Dicaprylate/Dicaprate | 2,0 % |
| Soya-Isoflavon-Aglykon/Liposomen-Wirkstoff (0,2 % Aglykone) | 5,0 % |
| PEG-60 Hydrogenated Castor Oil | 1,3 % |
| Konservierungsmittel, Natronlauge, Parfum, Aqua | ad 100,0 % |

### 11. Akne-Tinktur

| | |
|---|---|
| Soya-Isoflavon-Aglykon-Wirkstoff in Alkohol (2 % Aglykone) | 20,0 % |
| PEG-60 Hydrogenated Castor Oil | 10,0 % |
| Konservierungsmittel, Aqua | ad 100,0 % |

### 12. Whitening-Creme (Aufheller)

| | |
|---|---|
| Ethylhexyl Methoxycinnamate | 6,5 % |
| Caprylic/Capric Triglyceride | 4,0 % |
| *Arctostaphylos Uva Ursi* | 2,0 % |
| Glycerin | 2,0 % |
| Cl 77891 | 2,0 % |
| Butyl Methoxydibenzoylmethane | 1,5 % |
| Dimethicone | 1,5 % |
| PEG-20 Methyl Glucose Sesquistearate | 1,2 % |
| Soya-Isoflavon-Aglykon/Liposomen-Wirkstoff (0,2 % Aglykone) | 5,0 % |
| Methyl Glucose Sesquistearate | 1,0 % |
| Nylon-12 | 0,8 % |
| Cetyl Alcohol | 0,75 % |
| Stearyl Alcohol | 0,75 % |
| Konservierungsmittel, Parfum, Aqua | ad 100,0 % |

### 13. After-Shave-Balsam gegen Zeichen der Hautalterung

| | |
|---|---|
| *Hamamelis virginiana*-Extrakt | 3,0 % |
| PEG-20 Methyl Glucose Sesquistearate | 2,7 % |
| Glycerin | 2,0 % |
| Aluminium Starch Octenyl Succinate | 2,0 % |
| Oleyl Oleate | 2,0 % |
| Glucose | 2,0 % |
| Soya-Isoflavon-Aglykon/Liposomen-Wirkstoff (0,2 % Aglykone) | 5,0 % |
| Methyl Glucose Sesquistearate | 0,9 % |
| Mentyl Lactate | 0,4 % |
| Allantoin | 0,4 % |
| Konservierungsmittel, Parfum, Aqua | ad 100,0 % |

## Patentansprüche

1. Hautbehandlungsmittel mit einem Gehalt an mindestens einem Isoflavon-Aglykon in einer biologisch aktiven Form als Wirkstoff, **dadurch gekennzeichnet, dass** es in Kombination enthält:
- als Isoflavon-Aglykon Genistein in einer Konzentration von 1 bis 500 mg pro kg des Hautbehandlungsmittels; und
- einen wässrigen Algenextrakt.

2. Hautbehandlungsmittel nach Anspruch 1, **dadurch gekennzeichnet, dass** die Konzentration des Genistein 20 bis 100 mg pro kg des Hautbehandlungsmittel beträgt.

3. Hautbehandlungsmittel nach Anspruch 1 oder 2, **dadurch gekennzeichnet, dass** es als Algenextrakt einen Extrakt aus Algen der Gattung *Spirulina* enthält.

4. Hautbehandlungsmittel nach Anspruch 3, **dadurch gekennzeichnet, dass** es als Algenextrakt einen Extrakt von *Spirulina platensis* enthält.

5. Hautbehandlungsmittel nach einem der vorangehenden Ansprüche, **dadurch gekennzeichnet, dass** das Genistein in Liposomen eingebaut ist.

## Claims

1. Skin treatment compositions with a content of at least one isoflavone aglycone in a biologically active form as active component, **characterised in that** the cosmetic contains in a combination:
- as isoflavone aglycone genistein in a concentration from 1 to 500 mg per kg of the cosmetic and
- an aqueous algal extract.

2. Skin treatment compositions according to claim 1 **characterised in that** the concentration of genistein is from 20 to 100 mg per kg of the cosmetic.

3. Skin treatment compositions according to claim 1 or 2 **characterised in that** the algal extract is an extract of algae of the genus *Spirulina*.

4. Skin treatment compositions according to claim 3, **characterised in that** the algal extract is an extract of *Spirulina platensis*.

5. Skin treatment compositions according to one of the proceeding claims **characterised in that** the genistein being incorporated into liposomes.

## Revendications

1. Produit de soin pour la peau comprenant une teneur en au moins une isoflavone aglycone présente en tant que principe actif sous une forme biologiquement active, **caractérisé en ce qu'**il contient en combinaison :
- comme isoflavone aglycone, de la génistéine en une concentration allant de 1 à 500 mg par kg du produit de soin pour la peau ; et
- un extrait aqueux d'algue.

2. Produit de soin pour la peau selon la revendication 1, **caractérisé en ce que** la concentration de génistéine est comprise entre 20 et 100 mg par kg du produit de soin pour la peau.

3. Produit de soin pour la peau selon la revendication 1 ou 2, **caractérisé en ce qu'**il contient comme extrait d'algue, un extrait d'algue du genre Spirulina.

4. Produit de soin pour la peau selon la revendication 3, **caractérisé en ce qu'**il contient comme extrait d'algue, un extrait de Spirulina platensis.

5. Produit de soin pour la peau selon l'une des revendications précédentes, **caractérisé en ce que** la génistéine est intégrée dans des liposomes.
